# EUROPÄISCHE PATENTSCHRIFT

(11) **EP 1 776 153 B1**
(45) Veröffentlichungstag und Bekanntmachung des Hinweises auf die Patenterteilung: **31.10.2018**
(21) Anmeldenummer: 05774404.7
(22) Anmeldetag: 02.08.2005
(51) Int. Cl.: A61M 16/08

(54) **VORRICHTUNG ZUR ABLEITUNG VON ATEMGAS AUS DEM INNENBEREICH EINER ATEMMASKE, SOWIE UNTER EINSCHLUSS DIESER VORRICHTUNG GEBILDETE ATEMMASKENANORDNUNG AN SICH**
DEVICE FOR EVACUATING BREATHING GAS FROM THE INTERIOR OF A BREATHING MASK, AND BREATHING MASK ARRANGEMENT COMPRISING SAID DEVICE
DISPOSITIF POUR EVACUER DU GAZ RESPIRATOIRE DE L'INTERIEUR D'UN MASQUE RESPIRATOIRE, AINSI QU'ENSEMBLE MASQUE RESPIRATOIRE COMPRENANT CE DISPOSITIF

(30) Priorität: 02.08.2004 DE 102004037344; 01.10.2004 DE 102004048006
(43) Veröffentlichungstag der Anmeldung: 25.04.2007
(73) Patentinhaber: ResMed R&D Germany GmbH, 82152 Martinsried (DE)
(72) Erfinder: LANG, Bernd, 82166 Gräfelfing (DE); BIENER, Achim, 85445 Aufkirchen (DE)
(74) Vertreter: Vossius & Partner Patentanwälte Rechtsanwälte mbB
(86) Internationale Anmeldenummer: PCT/EP2005/008372
(87) Internationale Veröffentlichungsnummer: WO 2006/015772

(56) Entgegenhaltungen:
- EP-A- 1 138 340
- EP-A- 1 362 610
- DE-C1- 19 757 703
- US-A- 4 462 397
- US-A- 5 937 851
- US-A1- 2003 094 177

## Beschreibung

Die Erfindung bezieht sich auf eine Vorrichtung zur Ableitung von exhaliertem, d.h. mit CO₂ befrachtetem Atemgas aus einem Atemgasversorgungssystem. Weiterhin bezieht sich die Erfindung auch auf eine unter Einschluss einer derartigen Vorrichtung gebildete Atemmaskenanordnung.

Insbesondere zur Behandlung schlafbezogener Atmungsstörungen ist es bekannt, ein atembares Gas, beispielsweise Umgebungsluft einem Patienten auf einem Druckniveau zuzuführen, das zumindest phasenweise über dem Umgebungsdruck liegt. Hierdurch wird es möglich, im Bereich der oberen Atemwege einen als pneumatische Schienung bezeichneten Effekt herbeizuführen, durch welchen etwaigen Obstruktionen in diesem Bereich vorgebeugt werden kann. Die Zufuhr eines atembaren Gases auf einem über dem Umgebungsdruck liegenden Niveau kann neben der Behandlung schlafbezogener Atmungsstörungen auch anderweitig therapeutisch vorteilhaft sein.

Bei den bekannten Systemen erfolgt die Atemgaszufuhr unter Verwendung einer flexiblen Atemgasleitung und einer patientenseitig applizierten Atemmaske. Derartige Systeme zur Atemgaszufuhr werden im Falle des Therapiebedarfs von den betroffenen Personen üblicherweise während der gesamten Schlafphase ohne besondere Aufsicht getragen. Die zuverlässige Ableitung des im exhalierten Atemgas enthaltenen CO2 ist von besonderer Bedeutung.

Aus der auf die Anmelderin zurückgehenden US-Patentschrift US 6 112 745 ist ein zur Anordnung zwischen einer Atemmaske und einer Atemgasschlauchleitung vorgesehener Adapter bekannt der zwei lösbar zusammengefügte und um eine Adapter-Längsachse zueinander drehbare Rohrabschnitte umfasst, wobei die Rohrabschnitte derart bemessen sind, dass im Bereich der Fügestelle ein Ringspalt entsteht über welchen kontinuierlich verbrauchtes Atemgas abgeleitet werden kann und somit im Bereich der Atemmaske ein permanenter Atemgasaustausch ermöglicht wird.

Aus der ebenfalls auf die Anmelderin zurückgehenden WO 03-059427 ist eine Atemmaske bekannt, bei welcher eine Ableitung von teilweise verbrauchtem Atemgas aus dem Maskeninnenbereich durch Abströmöffnungen erfolgt, die sich in einem in Applikationsposition der Atemmaske den Nasenöffnungen eines Anwenders gegenüberliegenden Wandungsbereich der Atemmaske befinden.

Dokument US 5937851 zeigt eine Schwenk-Abgasleitung zur drehbaren Verbindung einer Patientenmaske mit einer Förderleitung. Das Design der Schwenk-Abgasleitung umfasst eine Auslassöffnung zur Abgabe CO2 beladener Luft. Eine Kammer, die durch einen Abstand zweier Teile gebildet ist, dient dabei der Lärmreduzierung. Die ausströmende Luft wird vom Patienten weg geleitet.

Dokument EP 1362610 offenbart eine Vorrichtung zur Ableitung von Atemgas und weist ein Innenteil und ein Außenteil auf. Das Innenteil und das Außenteil begrenzen gemeinsam mindestens bereichsweise mindestens einen Ausströmspalt. Das Innenteil ist aus einem Sockelsegment und einem Anschlußsegment ausgebildet. Das Sockelsegment und das Anschlußsegment sind von mindestens zwei Distanzelementen miteinander verbunden, zwischen denen sich ein Durchlaß zur Verbindung eines Innenraumes des Innenteiles mit dem Ausströmspalt erstreckt.

Die DE 197 57 703 betrifft eine Auslaßöffnung für Kohlendioxid, wobei die Auslaßöffnung mit einem Auslaßventil versehen ist, das einen definierten Strömungswiderstand aufweist und konstruktiv derart ausgestaltet ist, daß nur geringe Strömungsgeräusche entstehen sollen.

Mit den bekannten Ansätzen zur Ableitung von Atemgas wird das Ziel verfolgt, einen hinreichenden Abstrom des Atemgases geräuscharm und unter möglichst geringer Beeinträchtigung des Patienten sowie auch einer ggf. neben dem Patienten liegenden Person zu ermöglichen.

Der Erfindung liegt die Aufgabe zugrunde, Lösungen anzugeben, die bei der Ableitung von verbrauchtem Atemgas aus dem Überdruckbereich eines Atemgaszufuhrsystems weitere Vorteile bieten.

Diese Aufgabe wird gemäß einem ersten Aspekt der Erfindung gelöst durch eine Ableitungsvorrichtung zur Ableitung von Atemgas das mit CO2 befrachtet ist aus einem Druckbereich einer, eine Atemmaske umfassenden Anordnung zur Atemgaszufuhr auf einem über dem Umgebungsdruck liegenden Druckniveau, mit einer Rohrstutzenstruktur die einen Atemgaskanal definiert der sich zwischen einem schlauchseitigen Eingangsquerschnitt und einem maskenseitigen Ausgangsquerschnitt erstreckt und in einem zwischen dem Eingangsquerschnitt und dem Ausgangsquerschnitt liegenden Bereich mit einem Abströmmündungsabschnitt versehen ist über welchen das abzuführende Atemgas in die Umgebung gelangt, wobei in einem zwischen dem Abströmmündungsabschnitt und dem maskenseitigen Ausgangsquerschnitt des Atemgaskanals liegenden Bereich eine den Atemgaskanal umsäumende Doppelwandstruktur ausgebildet ist, zum Abgriff des aus einem Maskeninnenbereich abzuführenden Atemgases unter weitgehender Umgehung des Atemgaskanals.

Dadurch wird es auf vorteilhafte Weise möglich, auch bei geringen Atemgasdrücken eine angemessene Austauschwirkung in einer hinsichtlich des Profils der im Maskeninnenbereich herrschenden Strömung vorteilhaften Weise zu erreichen. In besonders vorteilhafter Weise wird erreicht, dass ggf. über den Bedarf in den Maskeninnenbereich eingeleitetes Atemgas keine Zuglufterscheinungen verursacht. Weiterhin ergeben sich auch hinsichtlich eines verringerten Totraumvolumens Vorteile.

Die Doppelwandstruktur ist vorzugsweise durch ein Hülsenelement gebildet das in einen Auswaschelementhauptkörper eingesetzt, oder auf diesen aufgesetzt ist. Das Hülsenelement und der Auswaschelementhauptkörper sind vorzugsweise aus einem sterilisierbaren Kunststoffmaterial gefertigt. Das Hülsenelement und der Auswaschelementhauptkörper sind vorzugsweise miteinander lösbar verbunden. Es ist möglich, zur Verbindung des Auswaschelementhauptkörpers und des Hülsenelementes Steck- oder Raststrukturen vorzusehen durch welche eine Koppelung der beiden Strukturen in einer geeigneten Stellung ermöglicht wird.

Gemäß einer besonders bevorzugten Ausführungsform der Erfindung sind das Hülsenelement und der Auswaschelementhauptkörper so ausgebildet, dass das Hülsenelement im Bereich des maskenseitigen Ausgangsquerschnitts über den Auswaschelementhauptkörper übersteht. Dadurch wird es möglich, das aus dem Innenbereich einer Atemmaske abzuführenden Gas in einem hinter dem in den Maskeninnenbereich vordringenden Endabschnitt des Zuleitungskanals liegenden Bereich abzugreifen und hierdurch eine besonders günstige Innenraumspülung zu erreichen.

Es ist möglich, im Bereich der Doppelwandstruktur Luftführungsstrukturen zu bilden, die einen geforderten Luftwiderstand ergeben. Diese Luftführungsstrukturen können durch Stege, insbesondere unter Bildung von Labyrinthstrukturen in an dem Hülsenelement und/oder dem Auswaschelementhauptkörper ausgebildet sein. Es ist möglich, diese Luftführungsstrukturen so auszubilden, dass über die Anordnung des Hülsenelementes relativ zu dem Auswaschelementhauptkörper der Strömungswiderstand und damit die abströmende Luftmenge einstellbar ist. Diese Stege oder Vorsprünge können so angeordnet sein, dass durch diese die Spaltweite des in der Doppelwandstruktur gebildeten Luftweges festgelegt ist.

Der Hauptkörper ist vorzugsweise mit einer Drehadapter- oder Drehstutzenstruktur versehen, zur drehbewegbaren Lagerung eines Drehstutzenelementes. Die Drehadapterstruktur weist vorzugsweise mehrere nachgiebige und integral mit dem Auswaschelementhauptkörper ausgebildete Schenkel mit Rastnasen auf. Die Schenkel bilden vorzugsweise Teil der Atemgaskanalwandung.

Die Doppelwandstruktur ist vorzugsweise so ausgebildet, dass diese einen Spalt mit einer radialen Spalthöhe im Bereich von 0,3 bis 3,2 mm definiert.

Hinsichtlich einer Atemmaskenanordnung wird die eingangs angegebenen Aufgabe auch gelöst durch eine Atemmaskenanordnung mit einem durch einen Maskenbasiskörper und einer Dichtlippeneinrichtung umsäumten Maskeninnenraum, wobei der Maskenbasiskörper einen Anschlussabschnitt aufweist, zur Aufnahme einer Rohrstutzenstruktur die einen Atemgaskanal definiert der sich zwischen einem schlauchseitigen Eingangsquerschnitt und einem maskenseitigen Ausgangsquerschnitt erstreckt und in einem zwischen dem Eingangsquerschnitt und dem Ausgangsquerschnitt liegenden Bereich mit einem Abströmmündungsabschnitt versehen ist über welchen das abzuführende Atemgas in die Umgebung gelangt, wobei in einem zwischen dem Abströmmündungsabschnitt und dem maskenseitigen Ausgangsquerschnitt liegenden Bereich eine den der Atemgasverlagerung dienenden Atemgaskanal umsäumende Doppelwandstruktur ausgebildet ist, zum Abgriff des abzuführenden Atemgases unter vollständiger, oder weitgehender Abtrennung des abgegriffenen Luftstromes von dem, der Atemgasverlagerung dienenden Kanalbereich der Rohrstutzenstruktur.

Vorzugsweise sitzt die Rohrstutzenstruktur im Bereich der Doppelwandstruktur in dem Anschlussabschnitt des Maskenbasiskörpers.

Unter der Definition "unter weitgehender Umgehung des Atemgaskanals" sind insbesondere Abgriffsansätze zu verstehen bei welchen sich ein Eintrittsbereich über welchen das abzuführende Gas dem vermittels der Doppelwandstruktur zufließenden Kanal (Bezugszeichen 12) zufließt, sich außerhalb des Hauptkanals befindet. Unter Verwendung der erfindungsgemäßen Doppelwandstruktur werden jedoch auch dann noch Vorteile erreicht wenn der Eintrittsspalt nicht mehr als 10% oder ca. 8mm von der maskenseitigen Mündungskante des Hauptkanals in den Hauptkanal hinein einwärts versetzt angeordnet ist.

Weitere Einzelheiten und Merkmale der Erfindung ergeben sich aus der nachfolgenden Beschreibung in Verbindung mit der Zeichnung. Es zeigt:
- **Fig. 1**: eine Schnittansicht durch eine erfindungsgemäße Vorrichtung zur Ableitung von mit CO₂ befrachtetem Atemgas;
- **Fig. 2**: eine Schnittansicht zur Erläuterung einer unter Verwendung der gemäß Fig. 1 ausgebildeten Atemmaskenanordnung;
- **Fig. 3a**: eine perspektivische Ansicht einer weiteren Variante einer erfindungsgemäßen Vorrichtung;
- **Fig. 3b**: eine Radialschnittansicht zur Erläuterung der bei der Variante nach Fig. 3a vorgesehenen Kanalquerschnitte;
- **Fig. 4a**: ebenfalls eine Radialschnittansicht zur Erläuterung des Prinzips der durch eine Doppelwandstruktur bereitgestellten Strömungskanäle;
- **Fig. 4b**: eine Radialschnittansicht zur Erläuterung einer Variante mit einem teilblockierten Kanalsegment zur Reduzierung des Strömungsquerschnittes;
- **Fig. 5**: eine Axialschnittansicht eines weiteren Ausführungsbeispieles einer erfindungsgemäßen Anordnung zur Ableitung von mit CO₂ befrachtetem Atemgas;
- **Fig. 6**: eine perspektivische Ansicht einer erfindungsgemäßen Atemaske mit einem gekröpften (Schlauchanschluss-) Leitungselement das einen integrierten CO2-Ableitungspfad bereitstellt;
- **Fig. 7**: eine Draufsicht auf die Atemmaske nach Figur 6 zur weiteren Illustration von konstruktiven Merkmalen der Atemmaske, insbesondere hinsichtlich des gekröpften Leitungselementes;
- **Fig. 8**: eine perspektivische Ansicht des mehrteilig ausgebildeten Leitungselementes der Atemmaske nach den Figuren 6 und 7.

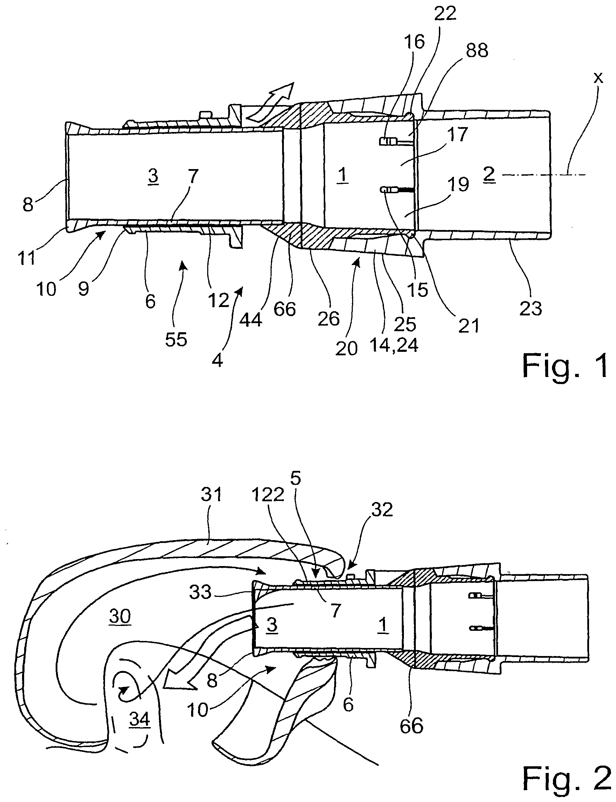

Bei der in Fig. 1 dargestellten Vorrichtung handelt es sich um eine mehrteilige Rohrstutzenstruktur, die einen zur Zuleitung eines atembaren Gases zu einer Atemmaske vorgesehenen Atemgaskanal 1 definiert. Dieser Atemgaskanal erstreckt sich zwischen einem schlauchseitigen Eingangsquerschnitt 2 und einem maskenseitigen Ausgangsquerschnitt 3. Diese Rohrstutzenstruktur ist mit einem Abströmmündungsabschnitt 4 versehen, über welchen abzuführendes Atemgas in die Umgebung gelangt. In einem sich zwischen dem Abströmmündungsabschnitt (4) und dem maskenseitigen Ausgangsquerschnitt (3) erstreckenden Bereich ist eine den Atemgaskanal (1) umsäumende Doppelwandstruktur (5) ausgebildet, die dem Abgriff eines aus dem in den Bereich einer Atemmaskenanordnung abzuführenden Atemgases dient, wobei die Doppelwandstruktur derart ausgebildet ist, dass dieser Abgriff im Bereich des maskenseitigen Ausgangsquerschnitts (3) oder einem diesem maskenseitigen Ausgangsquerschnitt 3 axial vorgelagerten Bereich erfolgt.

Bei diesem Ausführungsbeispiel ist die Doppelwandstruktur (5) aus einem Rohrstutzenabschnitt (6) und einem Hülsenelement (7) gebildet. Der Rohrstutzenabschnitt (6) bildet Teil eines Auswaschelement-Hauptkörpers (66), der aus einem thermoplastischen Kunststoffmaterial geformt ist und in welcher die zur Bereitstellung des Abströmmündungsabschnitts (4) erforderlichen Wandungen bereitgestellt sind.

Das Hülsenelement (7) ist bei diesem Ausführungsbeispiel derart in den Auswaschelement-Hauptkörper eingesetzt, dass in dem zwischen dem maskenseitigen Austrittsbereich (3) und dem Auswaschmündungsabschnitt (4) verlaufenden Kanalbereich jene Doppelwandstruktur (5) gebildet ist. Das Hülsenelement (7) und der Rohrstutzenabschnitt (6) sind derart dimensioniert, dass zwischen dem Außenumfangsbereich des Hülsenelementes (7) und der Innenumfangswandung des Rohrstutzenabschnitts (6) ein Zwischenraum verbleibt, der als Kanal fungiert, über welchen die aus dem Innenbereich der Atemmaske abzuleitende Luft zu dem Austrittsmündungsabschnitt (4) vordringen kann.

Bei diesem Ausführungsbeispiel ist das Hülsenelement (7) so ausgebildet, dass dieses lösbar in den Auswaschelement-Hauptkörper (66) einsetzbar ist. Das Hülsenelement (7) ist bei diesem Ausführungsbeispiel hinsichtlich seiner axialen Länge derart bemessen, dass dessen in den Maskeninnenbereich hineinragende Endumfangskante (8) axial über eine Endumfangskante oder Endstirnfläche (9) des Rohrstutzenabschnitts (6) übersteht. Hierdurch wird ein Axialversatz zwischen der maskenseitigen Mündung des Atemgaskanals (1) und dem Eintrittsspaltbereich (10) der Doppelwandstruktur (5) erreicht.

Das Hülsenelement (7) ist im Bereicht der Endumfangskante (8) mit einem Umfangswulst (11) versehen, durch welchen hinsichtlich der Spülung eines Maskeninnenraums noch weiter verbesserte Strömungsbedingungen geschaffen werden.

Der im Bereich der Doppelwandstruktur (5) gebildete Spalt ist derart dimensioniert, dass dieser einen dem Abfluss des abzuleitenden Atemgases zu dem Austrittsmündungsabschnitt (4) hinreichend drosselnden, jedoch nicht unzulässig hohen Strömungswiderstand aufweist. Im Bereich der Innenwandung des Rohrstutzenabschnitts (6) des Auswaschelement-Hauptkörpers (66) und/oder an der Außenumfangswandung des in den Auswaschelement-Hauptkörper (66) eingesetzten Hülsenelementes (7) können Strukturen z.B. Noppen oder kleine Einzelvorsprünge vorgesehen sein, durch welche ein gefordertes Mindestspaltmaß des in der Doppelwandstruktur (5) gebildeten Kanales (12) gewährleistet wird.

Im Bereich des Abströmmündungsabschnitts (4) ist vorzugsweise eine Abströmrampe (44) ausgebildet, durch welche das über den Kanal (12), dem Abströmmündungsabschnitt (4) zufließende Atemgas abgelenkt wird. Die Abströmrampe (44) ist vorzugsweise so ausgebildet, dass das austretende Atemgas noch über eine Wegstrecke von ca. 7 cm der Umfangswandung des Auswaschelement-Hauptkörper (66), bzw. einer auf diesem drehbar aufgesetzten Drehmuffe (14) und einem sich an diese wiederum anschließenden, hier nicht näher dargestellten Atemgasschlauch anschmiegt.

Der Auswaschelement-Hauptkörper (66) weist mehrere, durch Axialaussparungen (15, 16) voneinander getrennte Federschenkel (17, 18, 19) auf. Diese Federschenkel bilden Teil eines Drehlagerungsabschnitts (20) des Auswaschelement-Hauptkörpers (66), auf welchen die Drehmuffe (14) derart lösbar aufgesetzt ist, dass diese um die Längsachse (X) des Auswaschelement-Hauptkörpers (66) drehbar ist. An den Federschenkeln (17, 18, 19) sind Rastnasen (21, 22) ausgebildet, die in eine entsprechend hierzu komplementär in der Drehmuffe (14) ausgebildete Innenumfangsnut einschnappen können.

Die Drehmuffe (14) ist derart ausgebildet, dass diese einen zum Aufstecken eines flexiblen Atemgasschlauches vorgesehenen, schwach konisch ausgebildeten Schlauchaufsteckstutzen (23) und einen zum Aufsatz auf die Federschenkel (17, 18, 19), bzw. entsprechenden Abschnitt des Auswaschelement-Hauptkörpers (66) vorgesehenen Drehmuffenabschnitt (24) aufweist. Der Drehmuffenabschnitt (24) ist im Bereich seiner Außenumfangsfläche (25) schwach konisch ausgebildet und erweitert sich hierbei von einer an die Abströmrampe (44) angrenzenden Umfangswandung (26) auf den Außendurchmesser des auf den Schlauchaufsteckstutzen (23) aufzusteckenden Schlauches.

Obgleich aus dieser Darstellung nicht erkennbar, ist es möglich, die erfindungsgemäße Vorrichtung im Bereich des Auslaufmündungsabschnittes (4) so auszubilden, dass eine Ableitung des verbrauchten Atemgases an die Umgebung nicht über den gesamten Umfang des Auswaschelement-Hauptkörpers (66), sondern nur über einen ausgewählten Winkelbereich von beispielsweise 270° erfolgt, so dass eine Beaufschlagung des Nasenrücken-, Stirn- oder Kopfbereiches eines Maskenanwenders mit dem abgeleiteten Atemgas vermieden wird. Eine entsprechende Beeinflussung der Luftabströmung kann insbesondere durch am Auswaschelement-Hauptkörper (66) angebrachte Abschirmstrukturen oder auch durch lokales Blockieren des Kanals (12) erfolgen.

Die erfindungsgemäße Vorrichtung zur Ankoppelung eines Atemgasschlauches an eine Atemmaske unter gleichzeitiger Bereitstellung eines zur Ableitung von teilweise verbrauchten Atemgas vorgesehenen Spülweges ist in dieser Darstellung vergrößert gezeigt. Diese Vorrichtung ist vorzugsweise derart dimensioniert, dass der Innendurchmesser des Atemgaskanals in etwa 14 - 22 mm beträgt und die Gesamtlänge dieser Vorrichtung einschließlich des Aufsteckstutzens (23) der Drehmuffe beträgt vorzugsweise etwa 5,5 bis 10 cm.

Insbesondere über den Auswaschelement-Hauptkörper (66) können weitere, z. T. nachfolgend in Verbindung mit Detailskizzen noch angesprochene Strukturen bereit gestellt werden, durch welche weitere Kanäle, z.B. Druckmesskanäle oder Kanäle zur Sauerstoffzufuhr oder Luftsauerstoffgehaltsmessung bereit gestellt werden können.

In Fig. 2 ist in Form einer vereinfachten Schnittansicht dargestellt, wie eine - im wesentlichen wie vorangehend beschrieben ausgebildete - erfindungsgemäße Vorrichtung mit einem, einen Atemmaskeninnenraum (30) definierenden Maskenbasiskörper (31) gekoppelt werden kann. Bei diesem Ausführungsbeispiel ist der Maskenbasiskörper (31) aus einem elastomeren Material, insbesondere Silikonkautschuk gefertigt und umfasst einen in radialer Richtung elastisch weitbaren Anschlussabschnitt (32), in welchem die Vorrichtung nach Fig. 1 hinreichend festsitzend einsteckbar ist. Vorzugsweise ist die Geometrie des Maskenbasiskörpers (31) im Bereich des Anschlussabschnittes (32) sowie die Außengeometrie des Rohrstutzenabschnitts (6) des Auswaschelement-Hauptkörpers (66) so aufeinander abgestimmt, dass sich hierdurch eine hinsichtlich Abdichtung und Belastbarkeit hinreichende Koppelung der beiden Komponenten ergibt.

Bei der hier gezeigten Anordnung kann über den Atemgaskanal (1) zufließendes Atemgas über den maskenseitigen, hier durch das Hülsenelement (7) gebildeten Ausgangsquerschnitt (3) in den Atemmaskeninnenraum (30), wie durch das Pfeilsymbol P2 dargestellt, einfließen. Der Zufluss des Atemgases zu dem Atemmaskeninnenraum (30) kann, wie hier dargestellt, durch luftführende Strukturen (33) beeinflusst werden.

Die während einer Ausatemphase über die Nasenöffnungen (34) eines Anwenders in den Atemmaskeninnenraum (30) gelangende, CO₂ befrachtete Luft kann bei diesem Ausführungsbeispiel über einen hinter der Endumfangskante (8) liegenden Spaltbereich (10) in den durch die Doppelwandstruktur (5) gebildeten Kanal 12 abfließen. Durch den unter Verwendung des Hülsenelementes (7) zu den Nasenöffnungen (34) hin versetzten maskenseitigen Ausgangsquerschnitt des Atemgaskanals (1) und den, von diesen vorgeschobenen maskenseitigen Ausgangsquerschnitt (3) axial zurückversetzten Eintrittsspaltbereich (10) wird eine besonders vorteilhafte Spülung des Atemmaskeninnenraums (30) und hiermit verbunden eine vorteilhafte Ableitung des im Rahmen der Exhalationsphase aus den Nasenöffnungen (34) austretenden, CO₂ befrachteten Atemgases aus dem Atemmaskeninnenraum (30) erreicht.

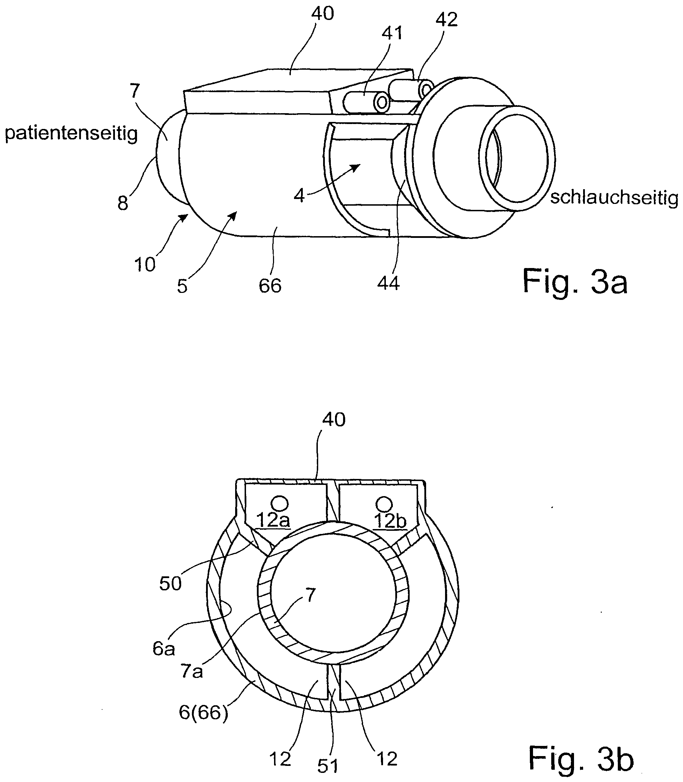

In Fig. 3a ist anhand einer perspektivischen Konzeptdarstellung eine Variante einer erfindungsgemäßen Vorrichtung gezeigt, bei welcher durch eine Doppelwandstruktur (5) ein axialer Versatz zwischen der zur Ableitung des CO₂ befrachteten Atemgases vorgesehenen Abgriffstelle (Eintrittsspaltbereich 10) und der Endumfangskante (8) des Hülsenelementes (7) erreicht wird. Bei diesem Ausführungsbeispiel ist der Auswaschelement-Hauptkörper (66) mit weiteren, Luftleitungskanäle bildenden Strukturen (40) versehen, durch welche Schlauchanschlusszapfen (41, 42) bereit gestellt werden, durch welche eine Atemgasdruckmessung oder auch eine Gaszu- oder - ableitung zu bzw. aus dem Innenbereich einer Atemmaske (vgl. Fig. 2) erfolgen kann. Auch bei diesem Ausführungsbeispiel ist im Bereich des Abströmmündungsabschnitts (4) eine Abströmrampe (44) vorgesehen, über welche das abströmende Atemgas vorteilhaft nach außen abgelenkt werden kann.

In Fig. 3b ist eine Axialschnittansicht der Vorrichtung nach Fig. 3a gezeigt. Wie aus dieser Ansicht erkennbar, ist das Hülsenelement (7) in den Rohrstutzenabschnitt (6) der Teil des Auswaschelement-Hauptkörpers (66) bildet, eingesetzt. Zwischen der Innenwandung (6a) und der Außenumfangswandung (7a) des Hülsenelementes (7) ist, wie vorangehend bereits beschrieben, ein Kanal (12) definiert, der bei diesem Ausführungsbeispiel durch Axialstege (50, 51, 52) längsgeteilt ist. Im Bereich der mit dem Auswaschelement-Hauptkörper (66) integral ausgebildeten Struktur (40) sind Kanäle (12a, 12b) gebildet, die ähnlich wie der zur Auswaschung dienende Kanal (12) mit dem Innenbereich einer Atemmaske kommunizieren und überdies in die in Verbindung mit Fig. 3a beschriebenen Schlauchanschlusszapfen (41, 42) münden. Der durch diese Doppelwandstruktur (5) gebildete Zwischenraum stellt insoweit ein Kanalsystem bereit, über welches eine Ableitung von CO₂ befrachtetem Atemgas, eine Druckmessung, eine zusätzliche Sauerstoffzufuhr, oder auch ein Luftabgriff, beispielsweise zur Analyse des Atemgases erfolgen kann.

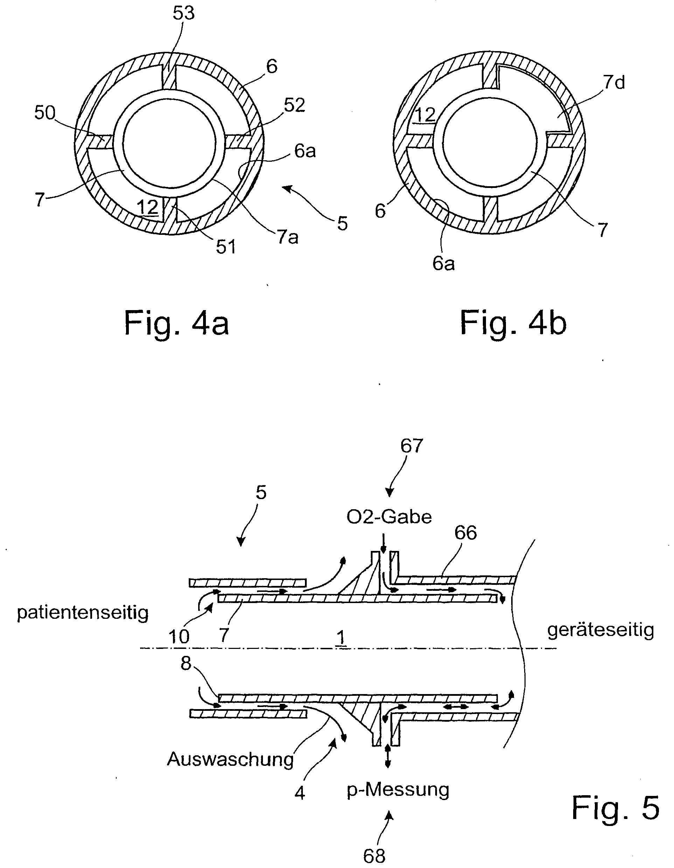

In Fig. 4a ist eine Variante dieser Doppelwandstruktur (5) dargestellt, bei welcher der zwischen den Wandungen (6a, 7a) bereit gestellte Zwischenraum bzw. -kanal (12) durch mehrere Längsstege (50, 51, 52, 53) in mehrere Längskanäle unterteilt ist. Das Hülsenelement (7) ist in den Rohrstutzenabschnitt (6) eingesetzt. Die genannten Stege (50, 53) sind bei diesem Ausführungsbeispiel integral mit dem Rohrstutzenabschnitt (6) ausgebildet.

Fig. 4b zeigt eine Variante, bei welcher das Hülsenelement (7) mit einem Blockiersteg (7d) versehen ist, durch welchen der für die Ableitung des CO₂ befrachteten Atemgases vorgesehene Querschnitt des Kanals (12) verringert werden kann. Es ist möglich, zur Festlegung des Atemgasabstromes das Hülsenelement (7) so auszubilden, dass dieses mehrere Sektionen des Kanals blockiert. Es ist auch möglich, an dem Hülsenelement Labyrinthe bildende Stegstrukturen auszubilden, die im Zusammenspiel mit der Innenwandung (6a) des Rohrstutzenabschnitts (6) Strömungswege mit gefordertem Strömungswiderstand bilden.

In Fig. 5 ist stark vereinfacht eine weitere Ausführungsform einer erfindungsgemäßen Vorrichtung gezeigt, bei welcher unter Verwendung einer Doppelwandstruktur (5) ein Axialversatz zwischen einem Eintrittsspaltbereich (10) und einem Austrittsmündungsabschnitt (4) erreicht wird, und zudem der Eintrittsspaltbereich (10) sich auf Höhe einer Endumfangskante (8) der den Atemgaskanal (1) definierenden Rohrstutzenstruktur befindet. Auch bei diesem Ausführungsbeispiel ist der Auswaschelement-Hauptkörper (66) mit Anschlussstrukturen (67, 68) versehen, über welche eine Gaszuleitung oder auch Gasableitung sowie insbesondere auch eine Druckmessung erfolgen kann. Die hierzu herangezogenen, in den auf einem gegenüber dem Umgebungsdruck erhöhten Druckniveau stehenden Bereich des Atemgaszufuhrsystems vorgesehenen Kanäle werden auch bei diesem Ausführungsbeispiel durch Doppelwandstrukturen gebildet, wie durch Einsatz eines Hülsenelementes (7) im Zusammenspiel mit einer Innenumfangswandung des Auswaschelement-Hauptkörpers (66) gebildet werden.

Die Erfindung ist nicht auf die vorangehend beschriebenen Ausführungsbeispiele beschränkt. Beispielsweise ist es auch möglich, den Auswaschelement-Hauptkörper (66) als Teil einer Hartschale einer Atemmaske auszubilden, so dass der Auswaschelement-Hauptkörper (66), insbesondere dessen zur Aufnahme der Drehmuffe (14) vorgesehener Drehlagerungsabschnitt (20) integraler Bestandteil der Atemmaskehartschale bilden. Das Hülsenelement (7) kann in den Auswaschelement-Hauptkörper (66) vom Innenraum des Schalenkörpers der Maske oder vorzugsweise auch vom schlauchseitigen Ende eingeschoben werden. Es ist auch möglich, das Hülsenelement (7) integral mit der Drehmuffe (14) auszubilden, d.h. das Hülsenelement (7) und die Drehmuffe (14) zu einem Teil zusammenzufassen.

Im Bereich des Abströmabschnitts (4) können auch von den vorangehend beschriebenen Ausführungsbeispielen abweichende, luftführende Strukturen, insbesondere Stege, Lamellen oder Kavernen angeordnet sein. Es ist auch möglich, im Bereich des Abströmabschnitts (4) Elastomerstrukturen vorzusehen, durch welche der Entstehung etwaiger Strömungsgeräusche weiter vorgebeugt werden kann. Das Hülsenelement (7) kann auch aus einem porösen Material gefertigt sein, so dass insbesondere zusätzlich zu dem Zutritt der abzuleitenden Luft über den Eintrittsspaltbereich (10), (Fig. 1) auch ein Luftzutritt in den Bereich des Abströmabschnitts oder des Kanals (12) durch die Wandung des Hülsenelementes (7) erfolgen kann.

Die erfindungsgemäße Vorrichtung kann alternativ oder in Kombination mit einer Drehmuffeneinrichtung auch mit einer Gelenkeinrichtung versehen sein, durch welche Kippbewegungen der Schlauchleitung relativ zu dem Auswaschelement-Hauptkörper (66) um Achsen ermöglicht werden, die im wesentlichen senkrecht zur Längsachse (X) des Auswaschelement-Hauptkörpers ausgerichtet sind. Die einzelnen Komponenten der erfindungsgemäßen Vorrichtung können insbesondere aus einem relativ steifen thermoplastischen Kunststoffmaterial gefertigt werden. Es ist auch möglich, insbesondere den Auswaschelement-Hauptkörper (66) aus einem hartelastischen Material zu fertigen.

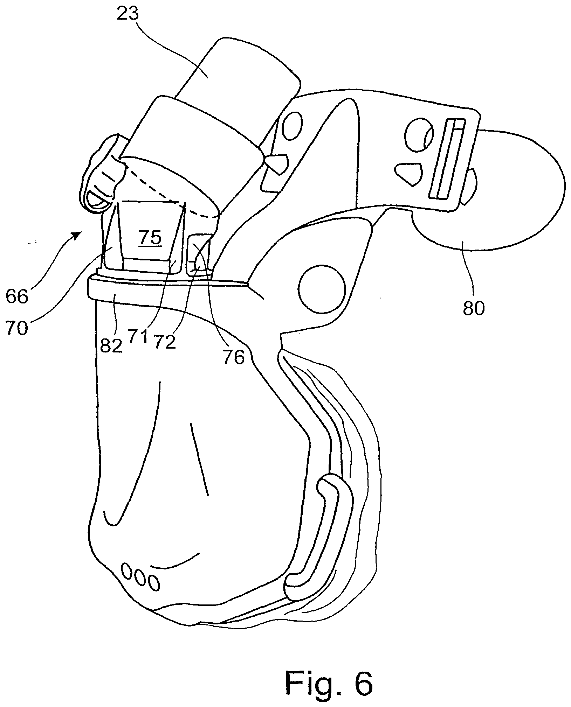

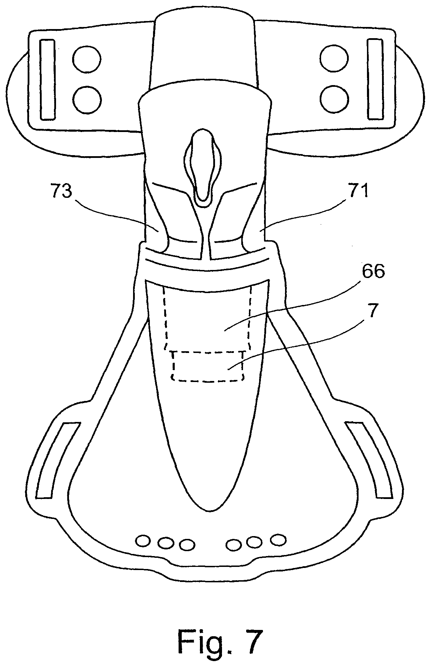

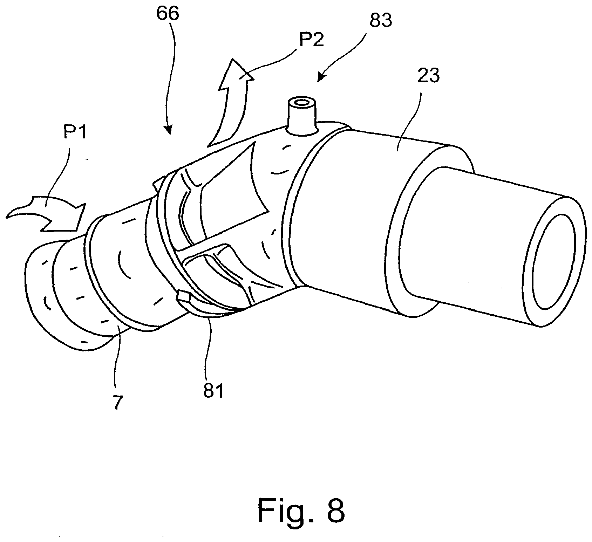

Für die Atemmaske gemäß den Figuren 6 und 7, sowie das Leitungselement nach Figur 8 gelten die vorangegangenen Ausführungen weitgehend sinngemäß. Für Einzelheiten und Komponenten der Atemmaske bzw. des Leitungselementes die als solche bereits in Verbindung mit den Figuren 1 bis 5 behandelt wurden, werden insoweit die gleichen Bezugszeichen verwendet z.T. ohne diese Komponenten nochmals anzusprechen.

Bei der Atemmaske nach den Figuren 6 und 7 ist der Auswaschelementhauptkörper 66 gekröpft ausgebildet. Der Kröpfungswinkel liegt im Bereich von 25 bis 60°, vorzugsweise bei 30°.

Im Bereich des Abströmmündungsabschnitts 4 sind vier Längsstege 71, 72, 73 (der vierte ist in dieser Darstellung nicht erkennbar). Der Auswaschelementhauptkörper 66 bildet auf seiner dem Anwender in Applikationsposition abgewandten Seite eine in Bezug auf die Anströmrichtung gesehen flache Rampenzone 75. Die Rampenzone 75 ist bei diesem Ausführungsbeispiel durch den Längssteg 70 geteilt und zudem durch den Steg 71 sowie den diametral gegenüberliegenden Steg 73 (Figur 7) eingefasst. Durch die Einfassung vermittels der Stege 71 und 73 wird eine vorteilhafte Luftführung erreicht.

Auf einer dem Anwender in Applikationsposition der Atemmaske zugewandten Sedite des Auswaschelementhauptkörpers 66 ist dieser mit einer steileren Rampenzone 76 versehen. Es ist möglich, Vorkehrungen zu treffen, derart, dass über diese Rampenzone ein geringerer Luftstrom abfließt als über die Rampenzone 75. Es ist auch möglich, hier eine Vollblockade zu realisieren.

Auf dem Auswaschelementhauptkörper 66 sitzt um seine Rohrachse herum drehbewegbar gelagert, der vorangehend bereits genannte Schlauchanschlussstutzen 23.

Die hier gezeigte Atemmaske zeichnet sich insbesondere dadurch aus, dass eine Ableitung des CO2 befrachteten Atemgases in einem Bereich der Maske erfolgt, der sich in Applikationsposition in etwa auf Höhe der Nasenwurzel des Anwenders, oder zumindest unterhalb des Bereichs liegt, in welchem die Maske am Patienten stirnseitig abgestützt ist (z.B. über die Stirnauflagepads 80).

Durch die besondere Gestaltung des Auswaschelementhauptkörpers 66 sowie die Verwendung des Hülsenelementes 7 (Figur 1) wird es möglich, eine Luftabführung bei geringem Totraumvolumen zu realisieren.

In Figur 8 ist in Form einer perspektivischen Darstellung der Auswaschelementhauptkörper 66 mit daran angesetztem Schlauchanschlussstutzen 23 sowie dem Hülsenelement 7 gezeigt. Der Auswaschelementhauptkörper 66 ist mit einer Befestigungsstruktur 81 versehen, über welche selbiger mit einem Rahmenabschnitt 82 (Figur 6) der Atemmaske koppelbar ist. Die Befestigungsstruktur 81 besteht hier aus zwei Bajonett-Flanken die mit einer in dem Rahmenabschnitt 82 entsprechend komplementär ausgebildeten Gegenstruktur in Eingriff bringbar sind.

In montiertem Zustand ragt der Auswaschelementhauptkörper 66 ähnlich wie in Figur 2 gezeigt mit seinem über den Rahmenabschnitt 82 hervorstehenden Teil, insbesondere auch mit dem Hülsenelement 7 in den Maskeninnenraum hinein. Eine Luftableitung (Pfeilsymbol P1) erfolgt über den zwischen der Außenumfangsfläche des Hülsenelementes 7 und der Innenfläche des Auswaschelementhauptkörpers 66 gebildeten Spaltbereich, der als solcher von dem eigentlichen Luftzuführungsweg abgeschirmt ist.

Die in den Spalt eindringende Luft fließt über die Rampenzone 75 (Pfeilsymbol P2) zur Umgebung hin ab.

Der Auswaschelementhauptkörper 66 ist in seinem nach außen weisenden Kniebereich mit einer Anschlussstruktur 83 versehen, die hier als Messschlauchanschlussstutzen ausgebildet ist. Über diese Struktur kann eine Druck- oder Gasmessung, und/oder auch eine aktive Sauerstoff- oder anderweitige Gaseinspeisung erfolgen. In den Figuren 6 und 7 ist diese Struktur mit einer Kappe abgedeckt.

## Patentansprüche

1. Ableitungsvorrichtung zur Ableitung von Atemgas, das mit CO2 befrachtet ist, aus einem Überdruckbereich einer, eine Atemmaske umfassenden und der Zufuhr eines Atemgases auf einem über dem Umgebungsdruck liegenden Druckniveau dienenden Anordnung, mit einer Rohrstutzenstruktur,
- die einen Atemgaskanal (1) definiert, der sich zwischen einem schlauchseitigen Eingangsquerschnitt (2) und einem maskenseitigen Ausgangsquerschnitt (3) erstreckt,
- und die in einem zwischen dem Eingangsquerschnitt (2) und dem Ausgangsquerschnitt (3) liegenden Bereich mit einem Abströmmündungsabschnitt (4) versehen ist, über welchen das abzuführende Atemgas in die Umgebung gelangt,
**dadurch gekennzeichnet, dass** in einem zwischen dem Abströmmündungsabschnitt (4) und dem maskenseitigen Ausgangsquerschnitt (3) des Atemgaskanals (1) liegenden Bereich eine den Atemgaskanal (1) umsäumende und einen außerhalb des Atemgaskanals (1) angeordneten Eintrittsspaltbereich (10) aufweisende Doppelwandstruktur (5) ausgebildet ist, zum Abgriff des aus einem Maskeninnenbereich (30) abzuführenden Atemgases.

2. Vorrichtung nach Anspruch 1, **dadurch gekennzeichnet, dass** die Doppelwandstruktur (5) unter Einbindung eines Hülsenelements (7) gebildet ist.

3. Vorrichtung nach Anspruch 2, **dadurch gekennzeichnet, dass** das Hülsenelement (7) in einen Rohrstutzenabschnitt (6) eines Auswaschelementhauptkörpers (66) eingesetzt ist.

4. Vorrichtung nach Anspruch 2, **dadurch gekennzeichnet, dass** das Hülsenelement (7) auf den Auswaschelementhauptkörper (66) aufgesetzt ist.

5. Vorrichtung nach wenigstens einem der Ansprüche 1 bis 4, **dadurch gekennzeichnet, dass** das Hülsenelement (7) und der Auswaschelementhauptkörper (66) so ausgebildet sind, dass das Hülsenelement (7) im Bereich des maskenseitigen Ausgangsquerschnitts (3) über den Auswaschelementhauptkörper (66) axial übersteht.

6. Vorrichtung nach wenigstens einem der Ansprüche 1 bis 5, **dadurch gekennzeichnet, dass** im Bereich der Doppelwandstruktur (5) Luftfuhrungsstrukturen ausgebildet sind, die einen geforderten Strömungswiderstand ergeben.

7. Vorrichtung nach wenigstens einem der Ansprüche 1 bis 6, **dadurch gekennzeichnet, dass** die Luftfuhrungsstrukturen als Labyrinthstrukturen ausgebildet sind.

8. Vorrichtung nach wenigstens einem der Ansprüche 1 bis 7, **dadurch gekennzeichnet, dass** das Hülsenelement (7) und der Auswaschelementhauptkörper (66) lösbar miteinander gekoppelt sind.

9. Vorrichtung nach wenigstens einem der Ansprüche 1 bis 8, **dadurch gekennzeichnet, dass** der Hauptkörper (66) mit einer Drehadapterstruktur versehen ist, zur drehbewegbaren Lagerung einer Drehmuffe (14).

10. Vorrichtung nach wenigstens einem der Ansprüche 1 bis 9, **dadurch gekennzeichnet, dass** die Doppelwandstruktur (5) einen Kanal (12) mit einer radialen Spalthöhe im Bereich von 0,3 bis 3,2 mm definiert.

11. Vorrichtung nach wenigstens einem der Ansprüche 1 bis 10, **dadurch gekennzeichnet, dass** im Zusammenspiel mit der Doppelwandstruktur (5) Stelleinrichtungen bewerkstelligt sind, zur Einstellung des Strömungswiderstandes.

12. Atemmaskenanordnung mit einem durch einen Maskenbasiskörper (31) und einer Dichtlippeneinrichtung definierten Maskeninnenraum (30), wobei der Maskenbasiskörper (31) einen Anschlussabschnitt (32) aufweist, mit aufnehmbare einer Rohrstutzenstruktur,
- die einen Atemgaskanal (1) definiert, der sich zwischen einem schlauchseitigen Eingangsquerschnitt (2) und einem maskenseitigen Ausgangsquerschnitt (3) erstreckt,
- und die in einem zwischen dem Eingangsquerschnitt (2) und dem Ausgangsquerschnitt (3) liegenden Bereich mit einem Abströmmündungsabschnitt (4) versehen ist, über welchen das abzuführende, CO₂-befrachtete Atemgas in die Umgebung gelangt, **dadurch gekennzeichnet, dass** in einem sich zwischen dem Abströmmündungsabschnitt (4) und dem maskenseitigen Ausgangsquerschnitt (3) liegenden Bereich eine den Atemgaskanal (1) umsäumende und einen außerhalb des Atemgaskanals (1) angeordneten Eintrittsspaltbereich (10) aufweisende Doppelwandstruktur (5) ausgebildet ist, zum Abgriff des aus einem Maskeninnenbereich (30) abzuführenden Atemgases .

13. Atemmaskenanordnung nach Anspruch 12, **dadurch gekennzeichnet, dass** die Rohrstutzenstruktur im Bereich der Doppelwandstruktur (5) in dem Anschlussabschnitt des Maskenbasiskörpers (31) sitzt.

14. Atemmaske für eine Atemmaskenanordnung nach Anspruch 12 oder 13.

15. Ableitungsvorrichtung zur Ableitung von Atemgas, das mit CO2 befrachtet ist, aus einem Überdruckbereich einer, eine Atemmaske umfassenden und der Zufuhr eines Atemgases auf einem über dem Umgebungsdruck liegenden Druckniveau zu einer Person dienenden Anordnung, mit einer Rohrstutzenstruktur,
- die einen Atemgaskanal (1) definiert der sich zwischen einem schlauchseitigen Eingangsabschnitt (2) und einem maskenseitigen Ausgangsabschnitt (3) erstreckt, und
- die in einem zwischen dem Eingangsabschnitt (2) und dem Ausgangsabschnitt (3) liegenden Bereich mit einem Abströmmündungsabschnitt (4) versehen ist, über welchen das abzuführende Atemgas in die Umgebung gelangt,
**dadurch gekennzeichnet, dass** in einem zwischen dem Abströmmündungsabschnitt (4) und dem maskenseitigen Ausgangsabschnitt (3) des Atemgaskanals (1) liegenden Bereich ein Hülsenelement (7) angeordnet ist, das den Atemgaskanal (1) umgibt und im Zusammenspiel mit dem Ausgangsabschnitt (3) eine Doppelwandstruktur (5) bildet, zum Abgriff des aus einem Maskeninnenbereich (30) abzuführenden Atemgases über einen Eintrittsspaltbereich (10), der dem maskenseitigen Endbereich des Ausgangsabschnitts (3) benachbart ist und außerhalb des Atemgaskanals (1) angeordnet ist.

16. Atemmaskeneinrichtung mit einem durch einen Maskenbasiskörper (31) und einer Dichtlippeneinrichtung definierten Maskeninnenraum (30), und einer Zuleitungskanaleinrichtung (1) zur Zuleitung von Luft in den Maskeninnenraum (30), wobei die Zuleitungskanaleinrichtung (1) einen in den Maskeninnenraum (30) vordringenden und an einer Mündungskante endenden Stutzen umfasst, **gekennzeichnet dadurch, dass** in einem gegenüber der Mündungskante zurückversetzten, außerhalb angeordneten Umgebungsbereich der Zuleitungskanaleinrichtung eine Ableitungsöffnung (4) vorgesehen ist, zur Ableitung von Atemgas aus dem Maskeninnenraum (30) an einer gegenüber der Mündungskante zurückversetzten Stelle.

## Claims

1. A diversion device for diverting breathing gas which is laden with CO2 from an overpressure region of an arrangement that includes a breathing mask and serves to deliver a breathing gas at a pressure level that is above the ambient pressure, having a tubular stub structure
- defining a breathing gas conduit (1) that extends between an inlet cross section (2) on the hose side and an outlet cross section (3) on the mask side,
- and which is provided, in a region located between the inlet cross section (2) and the outlet cross section (3), with an outflow orifice portion (4), by way of which the breathing gas to be discharged reaches the environment,
**characterized in that**, in a region located between the outflow orifice portion (4) and the outlet cross section (3) on the mask side of the breathing gas conduit (1), a double-walled structure (5) that surrounds the breathing gas conduit (1) and that comprises an inlet gap region (10) arranged outside of the breathing gas conduit (1), is configured for sampling the breathing gas to be discharged from a mask interior (30).

2. The device according to claim 1, **characterized in that** the double-walled structure (5) is formed with the inclusion of a sleeve element (7).

3. The device according to claim 2, **characterized in that** the sleeve element (7) is inserted into a tubular stub portion (6) of a main washing element body (66).

4. The device according to claim 2, **characterized in that** the sleeve element (7) is placed onto the main washing element body (66).

5. The device according to at least one of claims 1 through 4, **characterized in that** the sleeve element (7) and the main washing element body (66) are configured such that the sleeve element (7) protrudes axially past the main washing element body (66) in the region of the mask-side outlet cross section (3).

6. The device according to at least one of claims 1 through 5, **characterized in that** in the region of the double-walled structure (5), air guide structures are formed, which produce a required flow resistance.

7. The device according to at least one of claims 1 through 6, **characterized in that** the air guide structures are configured as labyrinth structures.

8. The device according to at least one of claims 1 through 7, **characterized in that** the sleeve element (7) and the main washing element body (66) are detachably coupled to one another.

9. The device according to at least one of claims 1 through 8, **characterized in that** the main body (66) is provided with a rotary adapter structure, for rotatably movably supporting a rotation cuff (14).

10. The device according to at least one of claims 1 through 9, **characterized in that** the double-walled structure (5) defines a conduit (12) with a radial gap height in the range from 0.3 to 3.2 mm.

11. The device according to at least one of claims 1 through 10, **characterized in that** in cooperation with the double-walled structure (5), adjusting devices are accomplished for adjusting the flow resistance.

12. A breathing mask arrangement having a mask interior (30) defined by a basic mask body (31) and a sealing lip device, wherein the basic mask body (31) has a connection portion (32) having a receivable tubular stub structure
- which defines a breathing gas conduit (1) that extends between an inlet cross section (2) on the hose side and an outlet cross section (3) on the mask side,
- and which is provided, in a region located between the inlet cross section (2) and the outlet cross section (3), with an outflow orifice portion (4) by way of which the CO2-laden breathing gas to be discharged reaches the environment, **characterized in that** in a region located between the outflow orifice portion (4) and the outlet cross section (3) on the mask side, a double-walled structure (5) that surrounds the breathing gas conduit (1) and that comprises an inlet gap region (10) arranged outside of the breathing gas conduit (1), is configured for sampling the breathing gas to be discharged from a mask interior region (30).

13. The breathing mask arrangement according to claim 12, **characterized in that** the tubular stub structure is seated in the region of the double-walled structure (5) in the connection portion of the mask base body (31).

14. A breathing mask for a breathing mask arrangement according to claims 12 or 13.

15. A diversion device for diverting breathing gas which is laden with CO2 from an overpressure region of an arrangement that includes a breathing mask and serves to deliver a breathing gas at a pressure level that is above the ambient pressure to a person, having a tubular stub structure
- defining a breathing gas conduit (1) that extends between an inlet portion (2) on the hose side and an outlet portion (3) on the mask side, and
- being provided, in a region located between the inlet portion (2) and the outlet portion (3), with an outflow orifice portion (4) by way of which the breathing gas to be discharged reaches the environment,
**characterized in that**, in a region located between the outflow orifice portion (4) and the outlet portion (3) on the mask side of the breathing gas conduit (1), a sleeve element (7) is located, which surrounds the breathing gas conduit (1) and, in cooperation with the outlet portion (3), forms a double-walled structure (5) for sampling the breathing gas to be discharged from a mask interior region (30) via an inlet gap region (10) that is adjacent to the mask-side end region of the outlet portion (3) and arranged outside of the breathing gas conduit (1).

16. A breathing mask means having a mask interior (30) defined by a mask base body (31) and a sealing lip device, and a delivery conduit means (1) for delivering air into the interior of the mask (30), wherein the delivery conduit means (1) comprises a stub extending into the interior of the mask (30) and ending at the orifice edge, **characterized in that** a diversion opening (4) is provided in a surrounding region of the delivery conduit means arranged outside and being recessed with respect to the orifice edge for diverting breathing gas from the interior of the mask (30) on a place being recessed with respect to the orifice edge.

## Revendications

1. Dispositif d'évacuation pour évacuer du gaz respiratoire qui est chargé en CO₂, d'une zone de surpression d'un ensemble comprenant un masque respiratoire et servant à l'apport d'un gaz respiratoire à un niveau de pression se trouvant au-dessus de la pression ambiante, avec une structure de tubulure,
- qui définit un canal de gaz respiratoire (1) qui s'étend entre une section transversale d'entrée (2) côté tuyau et une section transversale de sortie (3) côté masque,
- et qui est pourvue, dans une zone se trouvant entre la section transversale d'entrée (2) et la section transversale de sortie (3), d'une section d'orifice de décharge (4), par laquelle le gaz respiratoire à évacuer parvient dans l'environnement, **caractérisé en ce que** dans une zone se trouvant entre la section d'orifice de décharge (4) et la section transversale de sortie (3) côté masque du canal de gaz respiratoire (1) une structure à paroi double (5) présentant une zone de fente d'entrée (10) agencée en dehors du canal de gaz respiratoire (1) et entourant le canal de gaz respiratoire (1) est réalisée pour soutirer le gaz respiratoire à évacuer d'une zone intérieure de masque (30).

2. Dispositif selon la revendication 1, **caractérisé en ce que** la structure à double paroi (5) est formée en intégrant un élément de douille (7).

3. Dispositif selon la revendication 2, **caractérisé en ce que** l'élément de douille (7) est inséré dans une section de tubulure (6) d'un corps principal d'élément de lavage (66).

4. Dispositif selon la revendication 2, **caractérisé en ce que** l'élément de douille (7) est placé sur le corps principal d'élément de lavage (66).

5. Dispositif selon au moins l'une quelconque des revendications 1 à 4, **caractérisé en ce que** l'élément de douille (7) et le corps principal d'élément de lavage (66) sont réalisés de sorte que l'élément de douille (7) dépasse axialement dans la zone de la section transversale de sortie (3) côté masque du corps principal d'élément de lavage (66).

6. Dispositif selon au moins l'une quelconque des revendications 1 à 5, **caractérisé en ce que** des structures de guidage d'air sont réalisées dans la zone de la structure à double paroi (5), lesquelles produisent une résistance à l'écoulement exigée.

7. Dispositif selon au moins l'une quelconque des revendications 1 à 6, **caractérisé en ce que** les structures de guidage d'air sont réalisées en tant que structures de labyrinthe.

8. Dispositif selon au moins l'une quelconque des revendications 1 à 7, **caractérisé en ce que** l'élément de douille (7) et le corps principal d'élément de lavage (66) sont couplés de manière amovible l'un à l'autre.

9. Dispositif selon au moins l'une quelconque des revendications 1 à 8, **caractérisé en ce que** le corps principal (66) est pourvu d'une structure d'adaptateur rotatif pour le logement mobile en rotation d'un manchon rotatif (14).

10. Dispositif selon au moins l'une quelconque des revendications 1 à 9, **caractérisé en ce que** la structure à double paroi (5) définit un canal (12) avec une hauteur de fente radiale dans la plage de 0,3 à 3,2 mm.

11. Dispositif selon au moins l'une quelconque des revendications 1 à 10, **caractérisé en ce qu'**en interaction avec la structure à double paroi (5) des dispositifs de réglage sont réalisés pour le réglage de la résistance à l'écoulement.

12. Ensemble de masque respiratoire avec un espace intérieur de masque (30) défini par un corps de base de masque (31) et un dispositif de lèvre étanche, dans lequel le corps de base de masque (31) présente une section de raccordement (32) avec une structure de tubulure recevable,
- qui définit un canal de gaz respiratoire (1) qui s'étend entre une section transversale d'entrée (2) côté tuyau et une section transversale de sortie (3) côté masque,
- et qui est pourvue dans une zone se trouvant entre la section transversale d'entrée (2) et la section transversale de sortie (3) d'une section d'orifice de décharge (4), par laquelle le gaz respiratoire chargé en CO₂ à évacuer parvient dans l'environnement, **caractérisé en ce que** dans une zone se trouvant entre la section d'orifice de décharge (4) et la section transversale de sortie (3) côté masque une structure à double paroi (5) présentant une zone de fente d'entrée (10) agencée en dehors du canal de gaz respiratoire (1) et entourant le canal de gaz respiratoire (1) est réalisée pour soutirer le gaz respiratoire à évacuer d'une zone intérieure de masque (30).

13. Ensemble de masque respiratoire selon la revendication 12, **caractérisé en ce que** la structure de tubulure loge dans la zone de la structure à double paroi (5) dans la section de raccordement du corps de base de masque (31).

14. Masque respiratoire pour un ensemble de masque respiratoire selon la revendication 12 ou 13.

15. Dispositif d'évacuation pour l'évacuation de gaz respiratoire qui est chargé en CO₂ d'une zone de surpression d'un ensemble comprenant un masque respiratoire et servant à l'apport d'un gaz respiratoire à un niveau de pression se trouvant au-dessus de la pression ambiante à une personne, avec une structure de tubulure,
- qui définit un canal de gaz respiratoire (1) qui s'étend entre une section d'entrée (2) côté tuyau et une section de sortie (3) côté masque,
- et qui est pourvue dans une zone se trouvant entre la section d'entrée (2) et la section de sortie (3) d'une section d'orifice de décharge (4), par laquelle le gaz respiratoire à évacuer parvient dans l'environnement,
**caractérisé en ce qu'**un élément de douille (7) est agencé dans une zone se trouvant entre la section d'orifice de décharge (4) et la section de sortie (3) côté masque du canal de gaz respiratoire (1), lequel entoure le canal de gaz respiratoire (1) et forme en interaction avec la section de sortie (3) une structure à double paroi (5), pour soutirer le gaz respiratoire à évacuer d'une zone intérieure de masque (30) sur une zone de fente d'entrée (10) qui est contiguë à la zone d'extrémité côté masque de la section de sortie (3) et est agencée en dehors du canal de gaz respiratoire (1).

16. Dispositif de masque respiratoire avec un espace intérieur de masque (30) défini par un corps de base de masque (31) et un dispositif de lèvre étanche, et un dispositif de canal d'amenée (1) pour l'amenée d'air dans l'espace intérieur de masque (30), dans lequel le dispositif de canal d'amenée (1) comporte une tubulure dépassant dans l'espace intérieur de masque (30) et se terminant sur une arête d'orifice, **caractérisé en ce que** dans une zone environnante agencée en dehors, retirée par rapport à l'arête d'orifice du dispositif de canal d'amenée une ouverture d'évacuation (4) est prévue, pour l'évacuation de gaz respiratoire de l'espace intérieur de masque (30) à un endroit retiré par rapport à l'arête d'orifice.
